(19)

Europäisches
Patentamt
European
Patent Office
Office européen
des brevets

(11) **EP 4 129 083 A1**

(12) **EUROPEAN PATENT APPLICATION**
published in accordance with Art. 153(4) EPC

(43) Date of publication:
**08.02.2023 Bulletin 2023/06**

(21) Application number: **21776051.1**

(22) Date of filing: **24.03.2021**

(51) International Patent Classification (IPC):
**A23L 9/20** (2016.01)          **A23D 7/00** (1990.01)
**A23D 7/005** (1995.01)

(52) Cooperative Patent Classification (CPC):
**A23D 7/00; A23D 7/005; A23L 9/20**

(86) International application number:
**PCT/JP2021/012145**

(87) International publication number:
**WO 2021/193689 (30.09.2021 Gazette 2021/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.03.2020 JP 2020055223**

(71) Applicant: **Fuji Oil Holdings Inc.
Izumisano-shi, Osaka 598-8540 (JP)**

(72) Inventor: **IKENAGA, Naoya
Izumisano-shi, Osaka 598-8540 (JP)**

(74) Representative: **Dennemeyer & Associates S.A.
Postfach 70 04 25
81304 München (DE)**

(54) **HIGH-PROTEIN FOAMABLE OIL-IN-WATER EMULSION USING PLANT PROTEIN**

(57)     An objective of the present invention is to solve protein deficiency of elderly people who tend to fail to take sufficient protein only from their daily diet, by providing a plant protein-containing high-protein foamable oil-in-water emulsion used in bread, confectionery, and others. The present invention has been accomplished by finding that a plant protein-containing high-protein foamable oil-in-water emulsion which includes 3 to 15% by weight of plant protein can be obtained by containing 25 to 35% by weight of fats and oils constituting the foamable oil-in-water emulsion, in which fats and oils having a slip melting point of 33°C or lower is 50% by weight or more of the entire fats and oils.

EP 4 129 083 A1

**Description**

TECHNICAL FIELD

The present invention relates to a plant protein-containing high-protein foamable oil-in-water emulsion used in bread, confectionery, and others, which can solve protein deficiency of elderly people who tend to fail to take sufficient protein only from their daily diet.

BACKGROUND ART

In an effort underway to seek a sustainable world, there is an increased social sense of crisis concerning such problems as "shortage of food resources" and "environment".

Among others, food (especially, animal protein sources) and water resources are predicted to run short because of world population growth.

As a countermeasure to the social issues of "shortage of food resources" and "environment," which are caused associated with the above-described population growth, a solution through plant food raw materials is attempted.

Especially, soybean, pea, and others are considered as one of effective solutions to the human social issues.

This is because soybean, pea, and others can be cultivated in large amounts with minimal amounts of fertilizer and water, compared to animal protein sources, in a wide range of regions from cold climate areas to hot-arid areas.

On the other hand, our human bodies are largely constituted by, excluding water, protein and lipid.

Especially, protein is an important nutrient that is essential for any tissues, such as bones and hairs as well as muscles, organs, and skins.

Some Japanese take a sufficient amount of protein, which is 1 g/1 kg in weigh/day as a rough standard of a recommended amount. On the other hand, others currently have protein deficiency because of excessive dieting, skipping breakfast, eating less with aging, and the like.

Furthermore, sports population is rising with growing interest in health, and some others, who need protein in an amount equal to or more than the recommended amount because of increased exercise, do not take a sufficient amount of protein in some cases.

Especially, elderly people, who take insufficient protein, are susceptible to a state called "frail" (weak) such as muscle deterioration, resulting in a decrease in motor and cognition functions to have the risk of progressing to a state in which nursing care is required. Thus, the importance of protein is further increasing in rapidly-aging Japan.

Under such circumstances, an effort is made to achieve high protein in various foods such as protein products of, not only powder type, but also drink type or formed type which can be simply taken, such that elderly people can easily take more protein.

On the other hand, confectionery bread materials used in tasty bread, confectionery, and others are all not "high protein". Therefore, it is considered that if there is an opportunity to efficiently take protein by a snack or others other than diet, protein deficiency of elderly people, who lose appetite and tend to fail to take sufficient protein only from their daily diet, can be solved.

For example, Patent Document 1 discloses a "soybean milk whipping cream", but it is not high protein.

On the other hand, Patent Document 2 discloses a "high-protein and low-oil oil-in-water emulsion for whipping cream", but it essentially contains animal protein.

Also, Patent Document 3 discloses a high-protein low-fat cream that contains 25 to 44% of milk fat and 2.5 to 20% of milk protein, but it also essentially contains animal protein.

Therefore, a high-protein whipping cream containing plant protein has not been produced.

CITATION LIST

PATENT LITERATURE

Patent Document 1: JP-A-60-153757
Patent Document 2: JP-A-8-256717
Patent Document 3: JP-A-6-54648

SUMMARY OF THE INVENTION

PROBLEMS TO BE SOLVED BY THE INVENTION

An object of the present invention is to obtain a plant protein-containing high-protein foamable oil-in-water emulsion, which is used in bread, confectionery, and others.

SOLUTION TO THE PROBLEMS

The present inventor intensively conducted research for solving the above-described problems and accomplished the present invention by finding that a plant protein-containing high-protein foamable oil-in-water emulsion which includes 3 to 15% by weight of plant protein can be obtained by containing 25 to 35% by weight of fats and oils constituting the foamable oil-in-water emulsion, in which fats and oils having a slip melting point of 33°C or lower is 50% by weight or more of the entire fats and oils.

That is, the present invention is:

(1) a plant protein-containing high-protein foamable oil-in-water emulsion including 3 to 15% by weight of plant protein and 25 to 35% by weight of fats and oils, in which fats and oils having a slip melting point of 33°C or lower is 50% by weight or more of the entire fats and oils; and
(2) the plant protein-containing high-protein foamable oil-in-water emulsion described in (1), further including plant milk.

EFFECTS OF THE INVENTION

The present invention can provide a plant protein-containing high-protein foamable oil-in-water emulsion, used in bread, confectionery, and others, and can solve protein deficiency of elderly people who tend to fail to take sufficient protein only from their daily diet.

BEST MODE FOR CARRYING OUT THE INVENTION

Hereinafter, specific embodiments of the present invention will be described.

(Plant protein)

In the plant protein-containing high-protein foamable oil-in-water emulsion of the present invention, the plant protein refers to protein derived from plant raw materials.
Examples of the plant raw materials include: burdock, beet, carrot, Japanese radish, turnip, sweet potato, cassava, taro, dasheen, lotus root, and potato, as root vegetables; asparagus and bamboo shoot, as stem vegetables; and cabbage, onion, spinach, and lettuce, as leaf vegetables. Further examples include fruit vegetables and flower vegetables. Examples of the fruit vegetables include: rice and corn as cereal crops; red bean, mung bean, common bean, pea, black-eyed pea, broad bean, soybean, chickpea, peanut, and lentil, as legumes; and eggplant, tomatoes, bell pepper/paprika, squash, and cucumber, as other fruit vegetables. Examples of the flower vegetables include artichoke, oilseed rape, broccoli, and cauliflower.
Among the above-described plant raw materials, plant protein of legumes is preferably adopted in the present invention. More preferably, soybean protein, among the plant protein of legumes, is suitable in the present invention.
The foamable oil-in-water emulsion according to the present invention contains plant protein in an amount of 3 to 15% by weight, preferably 5 to 13% by weight, and more preferably 7 to 11% by weight.
When the plant protein is less than 3% by weight, the object of the present invention of efficiently taking protein is not achieved. When the plant protein exceeds 15% by weight, the viscosity of the oil-in-water emulsion increases, preparation tends to become difficult, and a phenomenon in which foaming is difficult can occur.
Also, in the present invention, use of animal protein is not precluded. Animal protein such as milk protein may be partially used for the purpose of achieving high protein.

(Fats and oils)

Examples of fats and oils used in the plant protein-containing high-protein foamable oil-in-water emulsion according to the present invention include plant fats and oils such as palm oil, coconut oil, palm kernel oil, soybean oil, rapeseed oil, sunflower seed oil, cottonseed oil, peanut oil, rice bran oil, corn oil, safflower oil, olive oil, kapok oil, sesame oil, and evening primrose oil, and medium chain fatty acid triglyceride. Also, when animal protein is used for the purpose of achieving high protein, use of animal fats and oils such as milk fat, beef tallow, and lard is not precluded. Usable fats and oils may be one or a mixture of the above-described fats and oils, or their hardened oil, fractionated oil, hardened and fractionated oil, fractionated and hardened oil, and processed fats and oils subjected to transesterification or the like.

The foamable oil-in-water emulsion according to the present invention contains fats and oils in an amount of 25 to 35% by weight and preferably 27 to 32% by weight. When the fats and oils are less than 25% by weight, foaming is inhibited, and a foamed state tends not to be stably maintained. When the fats and oils exceed 35% by weight, the viscosity of the oil-in-water emulsion increases, and preparation becomes difficult.

Also, the plant protein-containing high-protein foamable oil-in-water emulsion of the present invention contains fats and oils having a slip melting point of 33°C or lower in an amount of 50% by weight or more of the entire fats and oils. Examples of the fats and oils having a slip melting point of 33°C or lower include palm oil or its fractionated fats and oils, and coconut oil and palm kernel oil or their fractionated fats and oils as laurin-based fats and oils. The content of the fats and oils having a slip melting point of 33°C or lower is more preferably 53% by weight or more and further preferably 56% by weight or more. When the content of the fats and oils having a slip melting point of 33°C or lower is less than 50% by weight of the entire fats and oils, the melting point of the entire fats and oils increases, and melting in mouth tends to deteriorate under a high-protein condition.

It is noted that the slip melting point of fats and oils can be analyzed in accordance with Standard Methods for the Analysis of Fats, Oils and Related Materials (2.2.4.2-1996 Melting Point, Slip Melting Point).

(Plant milk)

In the present invention, the plant protein-containing high-protein foamable oil-in-water emulsion having a better flavor can be obtained by further containing plant milk to achieve high protein. The plant milk is extracts of nuts and seeds such as peanut, hazelnut, almond, cashew nut, macadamia nut, pistachio, coconut, sesame, and walnut or beans such as soybean, red bean, peanut, rice, barley, wheat, adlay, and chickpea. One or two or more of these are preferably contained in an amount of 5 to 60% by weight of the foamable oil-in-water emulsion.

In the present invention, soy milk, which is a plant milk of soybean, is preferably used. Fats and oils and protein derived from plant milk used in the present invention may be contained in the fats and oils and the protein of the foamable oil-in-water emulsion, respectively.

The plant protein-containing high-protein foamable oil-in-water emulsion of the present invention may contain saccharides as necessary. Examples of the saccharides include sucrose, fructose, glucose, lactose, maltose, invert sugar, trehalose, sugar alcohol, corn syrup, starch syrup, and dextrin. Example of the sugar alcohol include: monosaccharide alcohol such as erythritol, mannitol, sorbitol, and xylitol; disaccharide alcohol such as isomaltitol, maltitol, and lactitol; trisaccharide alcohol such as maltotriitol, isomaltotriitol, and panitol; tetrasaccharide or higher sugar alcohol such as oligosaccharide alcohol; reduced saccharified starch; and reduced hydrogenated starch.

The plant protein-containing high-protein foamable oil-in-water emulsion of the present invention preferably contains an emulsifier, thickening polysaccharides, and salts as necessary. Examples of the emulsifier include synthetic emulsifiers such as lecithin, monoglyceride, sorbitan fatty acid ester, propylene glycol fatty acid ester, polyglyceryl fatty acid ester, polyoxyethylene sorbitan fatty acid ester, and sucrose fatty acid ester. From these emulsifiers, one or two or more may be selected and appropriately used. Examples of the thickening polysaccharides include gellan gum, xanthan gum, locust bean gum, pullulan, guar gum, psyllium seed gum, water-soluble soybean saccharides, carrageenan, tamarind seed gum, and tara gum. From these thickening polysaccharides, one or two or more may be selected and appropriately used. Furthermore, examples of the salts include hexametaphosphate, secondary phosphate, sodium citrate, polyphosphate, and sodium bicarbonate. From these salts, one or a mixture of two or more may be used.

In addition, a flavor, a pigment, a preservative, and others may be optionally contained.

The plant protein-containing high-protein foamable oil-in-water emulsion according to the present invention can be obtained by mixing raw materials such as plant protein, fats and oils, water, and an emulsifier and thereafter performing preliminary emulsification, sterilization, and homogenization.

Sterilization is preferable in terms of preservability of the foamable oil-in-water emulsion. Specifically, various raw materials are preliminary emulsified at 60 to 70°C for 20 minutes (using a homomixer as an emulsification apparatus) and thereafter homogenized as necessary under the condition of 0 to 250 Kg/cm$^2$ (using a homogenizer as an emulsification apparatus).

Subsequently, the resultant product is subjected to ultra high temperature sterilization (UHT), homogenized again under the condition of 0 to 300 Kg/cm$^2$, cooled, and then aged for about 24 hours. Thereafter, the product is aseptically charged in a beverage paper container, a Scholle bag, or the like, thereby to obtain a plant protein-containing high-protein foamable oil-in-water emulsion which can be stored for a relatively long period through refrigeration.

According to the plant protein-containing high-protein foamable oil-in-water emulsion of the present invention, the overrun after foaming, under the foaming conditions described in Examples, is 80 to 200%, preferably 90 to 190%, and further preferably 100 to 180%.

When the overrun is excessively high, texture tends to become excessively light, less in cool sense, and poor in flavor.

When the overrun is excessively low, texture becomes excessively heavy, and a good flavor and a sense of melting in mouth are difficult to be obtained.

EXAMPLES

Hereinafter, the present invention will be more specifically described by illustrating examples. It is noted that the values in examples are based on weight, unless otherwise stated.

(Example 1)

An oil phase was prepared by adding, mixing, and dissolving 0.4 part of an emulsifier in 20 parts of palm fractionated fats and oils (slip melting point: 26°C) and 9 parts of laurin-based fats and oils (palm kernel oil/slip melting point: 28°C).

An aqueous phase was prepared by adding, mixing, and dissolving 5 parts of powdered soybean protein in 25.7 parts of water and 40 parts of plain soy milk while stirring by a homomixer.

The prepared oil phase and aqueous phase were stirred at 65°C for 30 minutes by a homomixer for preliminary emulsification, sterilized by a direct heating method at 144°C for 4 seconds using an ultra high temperature sterilizer (manufactured by Iwai Kikai Kogyo Co., Ltd.), homogenized at a homogenization pressure of 45 Kg/cm$^2$, and then immediately cooled to 5°C.

After cooling, the resultant product was aged for about 24 hours to obtain a foamable oil-in-water emulsion.

(Example 2)

An oil phase was prepared by adding, mixing, and dissolving 0.4 part of an emulsifier in 20 parts of palm fractionated fats and oils (slip melting point: 26°C) and 9 parts of laurin-based fats and oils (palm kernel oil/slip melting point: 28°C).

An aqueous phase was prepared by adding, mixing, and dissolving 5 parts of powdered pea protein in 25.7 parts of water and 40 parts of plain soy milk while stirring by a homomixer.

From the prepared oil phase and aqueous phase, a foamable oil-in-water emulsion was obtained in a similar manner to Example 1.

(Example 3)

An oil phase was prepared by adding, mixing, and dissolving 0.4 part of an emulsifier in 20 parts of palm fractionated fats and oils (slip melting point: 26°C) and 9 parts of laurin-based fats and oils (palm kernel oil/slip melting point: 28°C).

An aqueous phase was prepared by adding, mixing, and dissolving 4 parts of enzyme-treated powdered soybean protein in 26.7 parts of water and 40 parts of soy milk cream while stirring by a homomixer.

From the prepared oil phase and aqueous phase, a foamable oil-in-water emulsion was obtained in a similar manner to Example 1.

(Example 4)

An oil phase was prepared by adding, mixing, and dissolving 0.4 part of an emulsifier in 18 parts of palm fractionated fats and oils (slip melting point: 26°C) and 7 parts of laurin-based fats and oils (palm kernel oil/slip melting point: 28°C).

An aqueous phase was prepared by adding, mixing, and dissolving 9 parts of powdered soybean protein in 25.7 parts of water and 40 parts of plain soy milk while stirring by a homomixer.

From the prepared oil phase and aqueous phase, a foamable oil-in-water emulsion was obtained in a similar manner to Example 1.

(Example 5)

An oil phase was prepared by adding, mixing, and dissolving 0.4 part of an emulsifier in 16 parts of palm fractionated fats and oils (slip melting point: 26°C) and 4 parts of laurin-based fats and oils (palm kernel oil/slip melting point: 28°C).

An aqueous phase was prepared by adding, mixing, and dissolving 13 parts of powdered soybean protein in 26.7 parts of water and 40 parts of plain soy milk while stirring by a homomixer.

From the prepared oil phase and aqueous phase, a foamable oil-in-water emulsion was obtained in a similar manner to Example 1.

(Example 6)

An oil phase was prepared by adding, mixing, and dissolving 0.4 part of an emulsifier in 20 parts of palm fractionated fats and oils (slip melting point: 26°C) and 9 parts of laurin-based fats and oils (palm kernel oil/slip melting point: 28°C).

An aqueous phase was prepared by adding, mixing, and dissolving 7 parts of powdered soybean protein in 63.7 parts of water while stirring by a homomixer.

From the prepared oil phase and aqueous phase, a foamable oil-in-water emulsion was obtained in a similar manner to Example 1.

(Example 7)

An oil phase was prepared by adding, mixing, and dissolving 0.4 part of an emulsifier in 20 parts of palm fractionated fats and oils (slip melting point: 26°C) and 9 parts of laurin-based fats and oils (palm kernel oil/slip melting point: 28°C).

An aqueous phase was prepared by adding, mixing, and dissolving 7 parts of powdered pea protein in 63.7 parts of water while stirring by a homomixer.

From the prepared oil phase and aqueous phase, a foamable oil-in-water emulsion was obtained in a similar manner to Example 1.

(Example 8)

An oil phase was prepared by adding, mixing, and dissolving 0.4 part of an emulsifier in 15 parts of palm fractionated fats and oils (slip melting point: 26°C) and 14 parts of laurin-based fats and oils (palm kernel oil/slip melting point: 28°C).

An aqueous phase was prepared by adding, mixing, and dissolving 5 parts of powdered soybean protein in 25.7 parts of water and 40 parts of plain soy milk while stirring by a homomixer.

From the prepared oil phase and aqueous phase, a foamable oil-in-water emulsion was obtained in a similar manner to Example 1.

(Example 9)

An oil phase was prepared by adding, mixing, and dissolving 0.4 part of an emulsifier in 15 parts of palm fractionated fats and oils (slip melting point: 26°C) and 14 parts of laurin-based fats and oils (palm kernel oil/slip melting point: 34°C).

An aqueous phase was prepared by adding, mixing, and dissolving 5 parts of powdered soybean protein in 25.7 parts of water and 40 parts of plain soy milk while stirring by a homomixer.

From the prepared oil phase and aqueous phase, a foamable oil-in-water emulsion was obtained in a similar manner to Example 1.

(Comparative Example 1)

An oil phase was prepared by adding, mixing, and dissolving 0.4 part of an emulsifier in 13 parts of laurin-based fats and oils (palm kernel oil/slip melting point: 28°C) and 16 parts of laurin-based fats and oils (palm kernel oil/slip melting point: 34°C).

An aqueous phase was prepared by adding, mixing, and dissolving 5 parts of powdered soybean protein in 25.7 parts of water and 40 parts of plain soy milk while stirring by a homomixer.

From the prepared oil phase and aqueous phase, a foamable oil-in-water emulsion was obtained in a similar manner to Example 1.

(Comparative Example 2)

An oil phase was prepared by adding, mixing, and dissolving 0.4 part of an emulsifier in 9 parts of laurin-based fats and oils (palm kernel oil/slip melting point: 28°C) and 20 parts of laurin-based fats and oils (palm kernel oil/slip melting point: 34°C).

An aqueous phase was prepared by adding, mixing, and dissolving 5 parts of powdered soybean protein in 25.7 parts of water and 40 parts of plain soy milk while stirring by a homomixer.

From the prepared oil phase and aqueous phase, a foamable oil-in-water emulsion was obtained in a similar manner to Example 1.

(Comparative Example 3)

An oil phase was prepared by adding, mixing, and dissolving 0.4 part of an emulsifier in 14 parts of palm fractionated fats and oils (slip melting point: 26°C) and 3 parts of laurin-based fats and oils (palm kernel oil/slip melting point: 28°C).

An aqueous phase was prepared by adding, mixing, and dissolving 5 parts of powdered soybean protein in 37.7 parts of water and 40 parts of plain soy milk while stirring by a homomixer.

From the prepared oil phase and aqueous phase, a foamable oil-in-water emulsion was obtained in a similar manner to Example 1.

(Comparative Example 4)

An oil phase was prepared by adding, mixing, and dissolving 0.4 part of an emulsifier in 26 parts of palm fractionated fats and oils (slip melting point: 26°C) and 12 parts of laurin-based fats and oils (palm kernel oil/slip melting point: 28°C).

An aqueous phase was prepared by adding, mixing, and dissolving 5 parts of powdered soybean protein in 16.7 parts of water and 40 parts of plain soy milk while stirring by a homomixer.

From the prepared oil phase and aqueous phase, a foamable oil-in-water emulsion was obtained in a similar manner to Example 1.

(Comparative Example 5)

An oil phase was prepared by adding, mixing, and dissolving 0.4 part of an emulsifier in 16 parts of palm fractionated fats and oils (slip melting point: 26°C) and 4 parts of laurin-based fats and oils (palm kernel oil/slip melting point: 28°C).

An aqueous phase was prepared by adding, mixing, and dissolving 17 parts of powdered soybean protein in 22.7 parts of water and 40 parts of plain soy milk while stirring by a homomixer.

From the prepared oil phase and aqueous phase, a foamable oil-in-water emulsion was obtained in a similar manner to Example 1.

(Evaluation method of foamable oil-in-water emulsion)

•Viscosity

The viscosity of the foamable oil-in-water emulsion was measured by a B-type viscometer (manufactured by Brookfield Co., Model: LVDV-E) under the conditions of No.62, spindle, and 60 rpm.

The temperature of the foamable oil-in-water emulsion was measured at 7°C.

•Whipping time

One kilogram of the foamable oil-in-water emulsion was whipped by a Hobart mixer (manufactured by Hobart Corporation, Model: N-5) at speed 3 (300 rpm), and a time taken to reach an optimal foaming state was measured.

•Overrun

The overrun was calculated in the above-described optimal foaming state according to the following expression.

$$[(\text{Weight of oil-in-water emulsion at constant volume}) - (\text{weight of foamed}$$

$$\text{product after foaming at constant volume})]/(\text{weight of foamed product after foaming at}$$

$$\text{constant volume}) \times 100$$

•Flavor and melting in mouth:

The flavor and melting in mouth of the foamed oil-in-water emulsion were measured by a sensory test by 15 trained panelists.

In the sensory test, a rating was determined through evaluation based on the below-described five levels, and an average point of the 15 panelists was defined as an evaluation point of the foamable oil-in-water emulsion.

Flavor: good 5 to 1 bad
Melting in mouth: good 5 to 1 bad

The formulations and evaluation results of the foamable oil-in-water emulsions according to Examples 1 to 9 and Comparative Examples 1 to 5 are indicated in <Table 1> and <Table 2>.

| <Table 1> | Example 1 | Example 2 | Example 3 | Example 4 | Example 5 | Example 6 | Example 7 |
|---|---|---|---|---|---|---|---|
| Palm fractionated fats/oils (SMP: 26°C) | 20 | 20 | 20 | 18 | 16 | 20 | 20 |
| Laurin-based fats/oils (SMP: 28°C) | 9 | 9 | 9 | 7 | 4 | 9 | 9 |
| Laurin-based fats/oils (SMP: 34°C) | | | | | | | |
| Powdered soybean protein (*1) | 5 | | | 9 | 13 | 7 | |
| Powdered enzyme-treated soybean protein (*2) | | | 4 | | | | |
| Powdered pea protein (*3) | | 5 | | | | | 7 |
| Plain soy milk (*4) | 40 | 40 | | 40 | 40 | | |
| Soy milk cream (*5) | | | 40 | | | | |
| Water | 25.7 | 25.7 | 26.7 | 25.7 | 26.7 | 63.7 | 63.7 |
| Emulsifier | 0.4 | 0.4 | 0.4 | 0.4 | 0.4 | 0.4 | 0.4 |
| Total | 100 | 100 | 100 | 100 | 100 | 100 | 100 |
| | | | | | | | |
| Lipid | 30.2 | 30.2 | 34.3 | 26.2 | 21.2 | 29.0 | 29.0 |
| Ratio of fats/oils having SMP of ≤33°C | 96% | 96% | 84% | 95% | 94% | 100% | 100% |
| Plant protein | 5.9 | 5.6 | 5.7 | 9.4 | 12.8 | 6.0 | 5.5 |
| Carbohydrate | 0.9 | 0.8 | 0.5 | 1.1 | 1.2 | 0.3 | 0.1 |
| | | | | | | | |
| Liquid viscosity (cP) | 323 | 296 | 299 | 405 | 448 | 330 | 301 |

(continued)

| <Table 1> | Example 1 | Example 2 | Example 3 | Example 4 | Example 5 | Example 6 | Example 7 |
|---|---|---|---|---|---|---|---|
| Whipping time ('min"sec) | 1'43" | 1'49" | 1'51 | 2'17" | 2'40" | 2'01" | 2'09" |
| Overrun (%) | 170 | 166 | 167 | 182 | 189 | 170 | 169 |
| | | | | | | | |
| Flavor | 5 | 5 | 5 | 5 | 4 | 4 | 4 |
| Melting in mouth | 5 | 5 | 5 | 5 | 5 | 5 | 5 |
| | | | | | | | |
| Powdered soybean protein (*1) | Fujipro-F (soybean protein: 86.3%, carbohydrate: 4.0%) | | | | | | |
| Powdered enzyme-treated soybean protein (*2) | Fujipro-CLE (soybean protein : 86.3%, carbohydrate: 3.5%) | | | | | | |
| Powdered pea protein (*3) | NUTRALYS F85M (Roquette Freres, pea protein: 79%, carbohydrate: 1.0%) | | | | | | |
| Plain soy milk (*4) | Plain soy milk (lipid: 3%, protein: 4.4%, carbohydrate: 1.8%) (manufactured by Fuji Oil Co., Ltd.) | | | | | | |
| Soy milk cream (*5) | Soy milk cream (lipid: 13.3%, protein: 5.5%, carbohydrate: 0.9%) (manufactured by Fuji Oil Co., Ltd.) | | | | | | |

| \<Table 2\> | Example 8 | Example 9 | Comparative Example 1 | Comparative Example 2 | Comparative Example 3 | Comparative Example 4 | Comparative Example 5 |
|---|---|---|---|---|---|---|---|
| Palm fractionated fats/oils (SMP: 26°C) | 15 | 15 | | | 14 | 26 | 16 |
| Laurin-based fats/oils (SMP: 28°C) | 14 | | 13 | 9 | 3 | 12 | 4 |
| Laurin-based fats/oils (SMP: 34°C) | | 14 | 16 | 20 | | | |
| Powdered soybean protein (*1) | 5 | 5 | 5 | 5 | 5 | 5 | 17 |
| Powdered enzyme-treated soybean protein (*2) | | | | | | | |
| Powdered pea protein (*3) | | | | | | | |
| Plain soy milk (*4) | 40 | 40 | 40 | 40 | 40 | 40 | 40 |
| Soy milk cream (*5) | | | | | | | |
| Water | 25.7 | 25.7 | 25.7 | 25.7 | 37.7 | 16.7 | 22.7 |
| Emulsifier | 0.4 | 0.4 | 0.4 | 0.4 | 0.4 | 0.4 | 0.4 |
| Total | 100 | 100 | 100 | 100 | 100 | 100 | 100 |
| Lipid | 30.2 | 30.2 | 30.2 | 30.2 | 18.2 | 39.2 | 21.2 |
| Ratio of fats/oils having SMP of ≤33° | 96% | 50% | 43% | 30% | 93% | 97% | 94% |
| Plant protein | 5.9 | 5.9 | 5.9 | 5.9 | 5.9 | 5.9 | 16.3 |
| Carbohydrate | 0.9 | 0.9 | 0.9 | 0.9 | 0.9 | 0.9 | 1.4 |
| Liquid viscosity (cP) | 334 | 334 | 331 | 360 | 222 | 741 | 832 |
| Whipping time ('min"sec) | 1'33" | 1'51" | 2'23" | 2'44" | None | None | None |
| Overrun (%) | 162 | 183 | 199 | 207 | None | None | None |
| Flavor | 5 | 5 | 5 | 5 | - | - | - |

| <Table 2> | Example 8 | Example 9 | Comparative Example 1 | Comparative Example 2 | Comparative Example 3 | Comparative Example 4 | Comparative Example 5 |
|---|---|---|---|---|---|---|---|
| Melting in mouth | 5 | 4 | 3 | 2 | - | - | - |
| Powdered soybean protein (*1) | Fujipro-F (soybean protein: 86.3%, carbohydrate: 4.0%) | | | | | | |
| Powdered enzyme-treated soybean protein (*2) | Fujipro-CLE (soybean protein: 86.3%, carbohydrate: 3.5%) | | | | | | |
| Powdered pea protein (*3) | NUTRALYS F85M (Roquette Freres, pea protein: 79%, carbohydrate: 1.0%) | | | | | | |
| Plain soy milk (*4) | Plain soy milk (lipid: 3%, protein: 4.4%, carbohydrate: 1.8%) (manufactured by Fuji Oil Co., Ltd.) | | | | | | |
| Soy milk cream (*5) | Soy milk cream (lipid: 13.3%, protein: 5.5%, carbohydrate: 0.9%) (manufactured by Fuji Oil Co., Ltd.) | | | | | | |

**Claims**

1.  A plant protein-containing high-protein foamable oil-in-water emulsion comprising:

    3 to 15% by weight of plant protein; and
    25 to 35% by weight of fats and oils,
    wherein fats and oils having a slip melting point of 33°C or lower is 50% by weight or more of the entire fats and oils.

2.  The plant protein-containing high-protein foamable oil-in-water emulsion according to claim 1, further comprising plant milk.

<div style="text-align:center">**INTERNATIONAL SEARCH REPORT**</div>

| International application No. |
|---|
| PCT/JP2021/012145 |

**A.   CLASSIFICATION OF SUBJECT MATTER**
A23L 9/20(2016.01)i; A23D 7/00(2006.01)i; A23D 7/005(2006.01)i
FI: A23D7/00 508; A23D7/005; A23L9/20
According to International Patent Classification (IPC) or to both national classification and IPC

**B.   FIELDS SEARCHED**

Minimum documentation searched (classification system followed by classification symbols)
A23L9/20; A23D7/00; A23D7/005

| Documentation searched other than minimum documentation to the extent that such documents are included in the fields searched | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C.   DOCUMENTS CONSIDERED TO BE RELEVANT**

| Category* | Citation of document, with indication, where appropriate, of the relevant passages | Relevant to claim No. |
|---|---|---|
| X | WO 2019/189810 A1 (FUJI OIL HOLDINGS INC.) 03 October 2019 (2019-10-03) claims 2, 6, paragraphs [0039], [0062], [0066]-[0068], examples 12-17, comparative example 11 | 1-2 |
| Y | JP 2010-220484 A (KANEKA CORP.) 07 October 2010 (2010-10-07) paragraphs [0024], [0025], [0041]-[0045], [0066] | 1-2 |
| Y | JP 5-236896 A (KAO CORP.) 17 September 1993 (1993-09-17) paragraphs [0001], [0006] | 1-2 |
| Y | JP 2011-55752 A (TAIYO YUSHI CORP.) 24 March 2011 (2011-03-24) paragraphs [0001], [0028] | 1-2 |

☐ Further documents are listed in the continuation of Box C.          ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 31 May 2021 (31.05.2021) | 15 June 2021 (15.06.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

INTERNATIONAL SEARCH REPORT

Information on patent family members

International application no.

PCT/JP2021/012145

| Patent Documents referred in the Report | Publication Date | Patent Family | Publication Date |
|---|---|---|---|
| WO 2019/189810 A1 | 03 Oct. 2019 | (Family: none) | |
| JP 2010-220484 A | 07 Oct. 2010 | (Family: none) | |
| JP 5-236896 A | 17 Sep. 1993 | (Family: none) | |
| JP 2011-55752 A | 24 Mar. 2011 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60153757 A
- JP 8256717 A
- JP 6054648 A